# EUROPEAN PATENT APPLICATION

(11) **EP 4 289 928 A2**
(43) Date of publication of application: **13.12.2023**
(21) Application number: 23177825.9
(22) Date of filing: 06.06.2023
(51) Int. Cl.: C12M 1/34

(54) **SPERM SORTING DEVICE**

(30) Priority: 08.06.2022 US 202217835626
(71) Applicant: iPreg Incorporation, Taipei City 106429 (TW)
(72) Inventor: CHANG, Ching-Wen, 106429 Taipei City (TW); CHUNG, Chen-Yen, 106429 Taipei City (TW); HUANG, Chung-Hsien, 106429 Taipei City (TW); LI, Bor-Ran, 106429 Taipei City (TW)
(74) Representative: DTS Patent- und Rechtsanwälte Schnekenbühl und Partner mbB

(57) **Abstract**

A sperm sorting device (100) adapted to collaborate with a temperature controlling component (200) is provided. The sperm sorting device (100) includes an upper portion (110), a lower portion (120) and a porous layer (130). The upper portion (110) is formed with an upper chamber (111). The lower portion (120) is formed with a lower chamber (121). The upper chamber (111) and the lower chamber (121) are disposed vertically. The temperature controlling component (200) is adapted to be disposed outside the upper portion (110) or the lower portion (120) to make the temperature of the upper chamber (111) higher than the lower chamber (121). The porous layer (130) is disposed between the upper chamber (111) and the lower chamber (121). The porous layer (130) is adapted to allow sperms to pass through from the lower chamber (121) to the upper chamber (111) and adapted to form a temperature difference between the upper chamber (111) and the lower chamber (121).

## Description

### BACKGROUND OF THE INVENTION

### 1. Field of the Invention

The present disclosure relates to a sperm sorting device, and more particularly to a sperm sorting device introducing temperature difference in conjunction with swim-up methods.

### 2. Description of the Related Art

Infertility is a major health problem worldwide and is estimated to affect 8-12% of couples in the reproductive age group (Agarwal et al., Lancet 2021; 10271:319-333). Assisted reproductive technology (ART) such as *in-vitro* fertilization (IVF) and intracytoplasmic sperm injection (ICSI) has been widely used for infertility treatment.

While selecting highly motile and morphologically normal sperm is the obvious key to *in vitro* fertilization (IVF) and intracytoplasmic sperm injection (ICSI) success. The performance of current standard ART procedures, such as swim-up technique and density gradient separation, is not entirely satisfactory due to ROS generation and increasing the level of oxidative DNA damage in sperm cells (Punjabi et al., J Assist Reprod Genet. 2019; 36: 1413-21).

Horizontal microfluidic chips enable the use of small volumes for the separation of sperm into viable and motile sperm from non-viable or non-motile sperm. Only sperms with high motility could reach the contracted region of the channel against the flow field and be transported to the outlet. Such devices can be found, for example, in patent publications US 10532357, CN 102242055B, and US 10450545. However, the process is often a time-consuming task and can have severe volume restrictions.

The device disclosed in patent publication US 10,422,737 incorporates a filter to cause sperm to move against the filter and gravity to reach the outlet. However, when patients with low sperm concentration and/or low motility, the separation result of the low yield and lower quality after sorting need to be overcome. Especially, men's sperm has been decreased in number and getting worse in swimming for some time now.

Sperm thermotaxis is one of the mechanisms for selecting capacitated spermatozoa to fertilize the oocyte. Thermotaxis is dependent on a temperature gradient established within the fallopian tube. Guided by this gradient, capacitated mammalian spermatozoa can swim away from the utero-tubal junction towards the warmer temperature where the oocyte awaits. In a recent publication, spermatozoa selection by thermotaxis in mice and human possess higher DNA integrity and increased blastocyst production as well as live birth rate of ICIS (Pérez-Cerezales et al. Scientific Reports 2018, 8:2902.). In addition, a mild heat treatment of asthenozoospermic males increased the number of motile sperm and pregnancy rate (Küçük et al. J Assist Reprod Genet 2008, 25:235-238).

Current methods of sperm selection by thermotaxis were operating on a petri dish or microfluidic chip with a horizontal thermal gradient provided by a laboratory hot plate or resistive heater (Pérez-Cerezales et al., Scientific Reports 2018; 8:2902). Li et al. disclosed a horizontal temperature-gradient system for spermatozoa isolation (CN 108504563A). However, using the methods described above did not provide the throughput needed to meet IVF criteria. Furthermore, in a horizontal temperature gradient system, to establish an effective temperature difference, the hot and cold reservoirs need to be separated by a distance, resulting in space constraints for the system. Besides, there's no thermotaxis-based device to assist the sorting of motile spermatozoa on the market so far.

### BRIEF SUMMARY OF THE INVENTION

Aspects and advantages of embodiments of the present disclosure will be set forth in part in the following description, or may be learned from the description, or may be learned through practice of the embodiments.

An example aspect of the present disclosure is directed to a sperm sorting device adapted to collaborate with a temperature controlling component. The sperm sorting device includes an upper portion, a lower portion and a porous layer. The upper portion is formed with an upper chamber. The lower portion is formed with a lower chamber. The upper chamber and the lower chamber are disposed vertically, and the temperature controlling component is adapted to be disposed outside the upper portion or the lower portion to make the temperature of the upper chamber higher than the lower chamber. The porous layer is disposed between the upper chamber and the lower chamber, adapted to allow sperms to pass through from the lower chamber to the upper chamber and adapted to form a temperature difference between the upper chamber and the lower chamber.

Preferably, the upper portion includes a top plate disposed at a top side of the upper chamber. The temperature controlling component is adapted to transfer heat uniformly through the top plate to the upper chamber.

Preferably, the top plate has a lower surface and is formed with a through-hole disposed at a higher end of the lower surface, and varied height difference is formed between the lower surface and a bottom of the upper chamber.

Preferably, the lower surface is an incline.

Preferably, a cross-sectional area of the through-hole is less than 10% of an area of the lower surface.

Preferably, the upper portion is further formed with a collecting port communicating with the upper chamber.

Preferably, the upper portion is further formed with an injecting port communicating with the lower chamber.

Preferably, a material of the porous layer is selected from the group consisting of polycarbonate, polyvinyl alcohol, polypropylene, polytetrafluoroethylene, polystyrene, polyethylene terephthalate, polyvinylidene fluoride, polyamide, non-woven fabric and polyurethane.

Preferably, the porous layer is formed with a plurality of holes, and pore sizes of the plurality of holes are 8-20 mm.

Another example aspect of the present disclosure is directed to a temperature difference device adapted for the sperm sorting device. The temperature difference device includes a main body, a lid and a temperature controlling component. The main body is formed with a sorting device accommodating portion configured to accommodate at least a portion of the sperm sorting device. The lid is connected to the main body and adapted to be selectively opened relative to the main body. The temperature controlling component is disposed on the lid.

Preferably, the temperature difference device further includes a controlling unit electrically connected to the temperature controlling component.

Preferably, an outer wall of the lower chamber is convex outwards, and the sorting device accommodating portion is formed with a concaved portion configured to receive the outer wall.

Preferably, the sorting device accommodating portion is formed of a material with high thermal conductivity coefficient or formed with a heat dissipation hole.

Preferably, the temperature controlling component is a heat plate.

The foregoing summary is illustrative only and is not intended to be in any way limiting. In addition to the illustrative aspects, embodiments, and features described above, further aspects, embodiments, and features will become apparent by reference to the figures and the following detailed description.

### BRIEF DESCRIPTION OF THE FIGURES

FIG. 1 is a flow chart of a sperm sorting method adapted for a sperm sorting device according to some exemplary embodiments.
FIG. 2 is a perspective view of a sperm sorting device according to some exemplary embodiments.
FIG. 3 is a cross-sectional view of the sperm sorting device in FIG. 2 during sorting sperms.
FIG. 4 is a perspective view of a temperature difference device according to some exemplary embodiments.
FIG. 5 is an illustrative diagram showing the relationship between temperature difference and time by applying the sperm sorting method and a sperm sorting method without porous layer according to some exemplary embodiments.
FIG. 6 is an illustrative diagram showing the sperm concentration by applying the sperm sorting method with temperature difference and a sperm sorting method without temperature difference according to some exemplary embodiments.
FIG. 7 is an illustrative diagram showing the percentage of progressive sperms (PR) recovery rate by applying the sperm sorting method with temperature difference and a sperm sorting method without temperature difference according to some exemplary embodiments.
FIG. 8 is an illustrative diagram showing the percentage of progressive sperms (PR) before sorting, by applying the sperm sorting with temperature difference and a sperm sorting method without temperature difference according to some exemplary embodiments.
FIG. 9 is an illustrative diagram showing the average path velocity (VAP) of the sperms before sorting, by applying the sperm sorting method with temperature difference and a sperm sorting method without temperature difference according to some exemplary embodiments.
FIG. 10 is an illustrative diagram showing the straight-line velocity (VSL) of the sperms before sorting, by applying the sperm sorting method with temperature difference and a sperm sorting method without temperature difference according to some exemplary embodiments.
FIG. 11 is an illustrative diagram showing the curvilinear velocity (VCL) of the sperms before sorting, by applying the sperm sorting method with temperature difference and a sperm sorting method without temperature difference according to some exemplary embodiments.
FIG. 12 shows the sperm DNA fragmentation in processed fraction and immunostaining of spermatozoa with TUNEL assay before sorting and by applying the sperm sorting method with temperature difference according to some exemplary embodiments.
FIG. 13 shows the sperm DNA fragmentation percentage with TUNEL assay before sorting and by applying the sperm sorting method with temperature difference according to some exemplary embodiments.

### DETAILED DESCRIPTION OF THE INVETION

The aforementioned and other technical features, characteristics and effects of the present disclosure may be clearly presented by the detailed description of exemplary embodiments together with the attached figures. In addition, in the following embodiments, the same or similar components will use the same or similar reference numerals.

In addition, the methods, processes and steps disclosed by the embodiments are only illustrative and not intended to limit. Therefore, a person skilled in the art can appropriately increase, omit, modify or execute each method, process or step alone without departing from the spirit and the scope of the invention, unless the change results in timing or technical inconsistencies. Besides, the orders of each method, process or step can also be altered or adjusted.

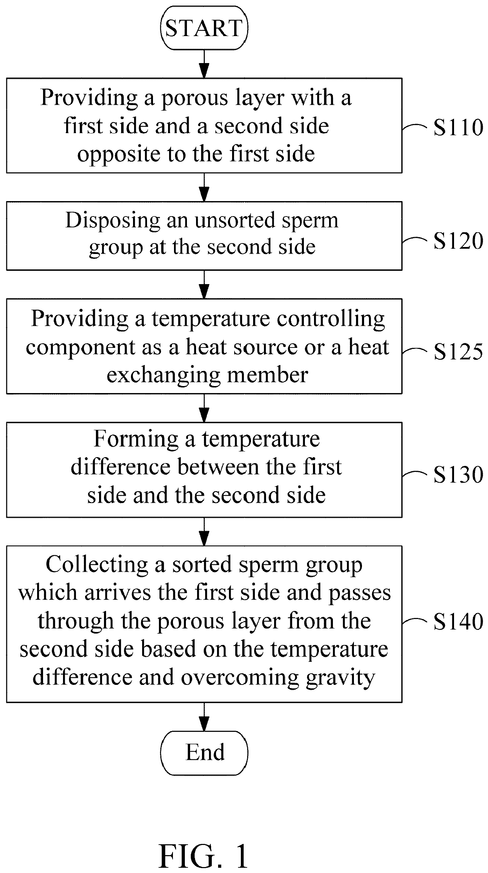

Refer to FIG. 1. The sperm sorting method introducing temperature difference and swim-up methods according to some exemplary embodiments includes the following steps: providing a porous layer with a first side and a second side opposite to the first side (step S110); disposing an unsorted sperm group at the second side (step S120); forming a temperature difference between the first side and the second side (step S130); and collecting a sorted sperm group which arrives the first side and passes through the porous layer from the second side based on the temperature difference and overcoming gravity (step S140).

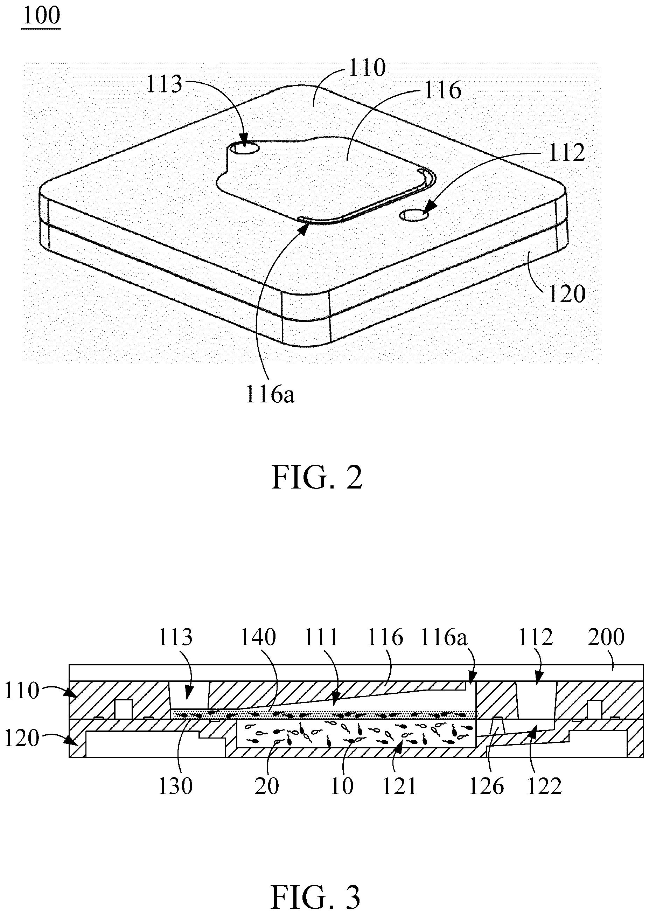

Refer to FIGS. 2 and 3. Specifically, the sperm sorting device 100 may include an upper portion 110 and a lower portion 120. The upper portion 110 and the lower portion 120 are adapted to be detachably combined with each other. However, in some embodiments, the upper portion 110 and the lower portion 120 may also be manufactured in an integrated molding process to form an integral structure. It is not limited by the present invention. Besides, the upper portion 110 may be formed with an upper chamber 111, and the lower portion 120 may be formed with a lower chamber 121. When the upper portion 110 and the lower portion 120 are combined, the upper chamber 111 and the lower chamber 121 are aligned with each other substantially, and a porous layer 130 is adapted to be disposed between the upper chamber 111 and the lower chamber 121. The porous layer 130 may be a biocompatible membrane, either hydrophobic or hydrophilic. In some embodiments, the porous layer 130 may be selected from a polycarbonate track-etched membrane or a polyvinyl alcohol track-etched membrane. Further, in some embodiments, the material of the porous layer 130 may also be selected from polymers such as polytetrafluoroethylene, polystyrene, polyethylene terephthalate, polyvinylidene fluoride, polyamide, non-woven fabric and polyurethane. Besides, the porous layer 130 is formed with a plurality of tiny holes. The pore sizes of these holes may be 8-20 mm. Thereby, a temperature difference can be formed between two opposite sides of the porous layer 130 while providing a heat source from the top plate 116, and the sperms to be sorted are allowed to move from one side of the porous layer 130 to another side thereof.

Moreover, the upper portion 110 may be further formed with an injecting port 112 and a collecting port 113. The injecting port 112 is configured to inject the sperm sample. The collecting port 113 communicates with the upper chamber 111 for collecting sorted sperm and injecting an optional buffer 140 during sorting. The lower portion 120 may be formed with a guiding portion 122. The guiding portion 122 is exemplary to be an inclined concave and communicate with the lower chamber 121. When the upper portion 110 and the lower portion 120 are joined, the injecting port 112 is aligned and communicates with the guiding portion 122. As shown in figure, the upper chamber 111 and the collecting port 113 are located at a first side of the porous layer 130, and the lower chamber 121 is located at a second side of the porous layer 130. By the configuration, user can inject an unsorted sperm group via the injecting port 112 and make it travel through the guiding portion 122 to the lower chamber 121 at the second side of the porous layer 130. Then, a temperature difference can be formed between the first side and the second side, while providing a heat source from the top plate 116. Since motile sperms have stronger thermotaxis behavior, the motile sperms 10 among the unsorted sperm group will overcome the gravity based on the temperature difference to pass through the porous layer 130 from the second side to the first side with higher temperature. The immotile sperms 20 tend to stay in the lower chamber 121 at the second side, and the sperm sorting is achieved.

In order to maximize the proportion of sperm motility dedicated to overcoming gravity rather than being consumed by relative motion friction with the porous layer 130, preferably, the porous layer 130 is disposed horizontally. That is, the first side and the second side are arranged vertically. Besides, in some embodiments, user can also deploy the buffer 140 into the upper chamber 111 at first side of the porous layer 130 through the collecting port 113. The buffer 140 may be a conventional sperm washing medium with bicarbonate, or an HEPES buffer to maintain the motility of the motile sperms 10 after passing through the porous layer 130.

Due to the moving characteristic of sperms to swim along the sidewall or towards obstacles, in some embodiments, the width between side walls of the guiding portion 122 may be designed to be gradually wider as approaching the lower chamber 121. Thereby, more sperms are allowed to travel towards the lower chamber 121. Besides, in some embodiments, the lower portion 120 further includes a guiding member 126. The guiding members 126 are exemplary to be two conical columns disposed on the guiding portion 122, and appropriate clearances are formed between the guiding members 126 and side walls of the guiding portion 122 and between the guiding member 126 themselves. By the configuration, the sperms can be promoted to travel along the side walls of the guiding portion 122 and the periphery of the guiding member 126, which further improves the chance of the sperms to advance to the lower chamber 121.

During the sperm sorting process, the porous layer 130 not only acts as a filtering member for the motile sperms 10 and the immotile sperms 20 to overcome the gravity, but also delays the thermal equilibrium between the upper chamber 111 and the lower chamber 121. On the other hand, in this embodiment, the method to form the temperature difference is exemplary to heat the buffer 140 at the first side of the porous layer 130 by a temperature controlling component 200, and the temperature of the upper chamber 111 is higher than that of the lower chamber 121. The temperature controlling component 200 may be a heat plate and disposed outside the upper portion 110 to avoid direct contact with the sperms in the upper chamber 111 and the buffer 140. Specifically, the upper portion 110 may include a top plate 116, and the temperature controlling component 200 is attached to the top plate 116. By the arrangement, the temperature controlling component 200 is capable of heating the upper chamber 111 through the top plate 116 uniformly rather than merely forming a regional temperature difference. In the embodiment, the temperature of the first side may be kept between 35-38°C, the temperature of the second side may be kept between 30-36°C, and the temperature of the first side is higher than the temperature of the second side. Preferably, the predetermined temperature mention above is slightly higher than the desired temperature of fluids in the chamber. By experiments, when the temperature difference is set to be 1-4°C, a better sorting result can be obtained without affecting the motility of the sperms, but it is not limited thereto.

It is noted that the temperature controlling component 200 is exemplified as a heating element for heating the upper chamber 111 in the embodiment. However, in some embodiments, the temperature controlling component 200 may be also a heat dissipation element for cooling the lower chamber 121, as shown in the step S125 in FIG. 1. For example, the temperature controlling component 200 may be a heat spreader and attached between an outer wall and a cooling source to cool the lower chamber 121 and form the temperature difference. In other words, any component that can indirectly contact the substances in the upper and lower chambers and form the temperature difference can be selected for the temperature controlling component 200.

In addition, the cooling method for the lower chamber 121 is not limited to indirect contact with a cooling source through a heat spreader. For example, cooling can be achieved through chemical means by applying thermal paste to the outer wall of the lower chamber 121, or through physical means by disposing cooling components such as fans outside the lower chamber 121. Another option is through materials with high thermal conductivity, such as metals, for the lower portion 120 to enhance heat dissipation. Alternatively, the platform that supports the lower portion 120 can be designed with multiple cooling holes for structural cooling. The present invention does not impose any limitations on these cooling methods.

On the other hand, when user injects the buffer 140 into the upper chamber 111, bubbles tend to be formed to affect the flow of sperms. Therefore, varied height difference is formed between a lower surface of the top plate 116 and a bottom of the upper chamber 111 in the embodiment. Specifically, the lower surface of the top plate 116 is formed as an incline, and a through-hole 116a is formed at a higher end of the incline. Thereby, during the injection of the buffer 140, the bubbles generated will rise along the mixed fluid and move along the incline, eventually being discharged through the through-hole 116a. However, in other embodiments, the lower surface of the top plate 116 may be also curved or stair-shaped, as long as it smoothly guides the bubbles towards the through-hole 116a.

It is noted that the dissolved oxygen in the fluid in the chamber is likely to generate free radicals which in turn damage the sperms, and the amount of the dissolved oxygen mentioned above is proportional to the contact area and contact time between the fluid and the air. Therefore, the area occupied by the through-hole 116a relative to the top plate 116 is suggested to be limited to avoid excessive contact area to the air. The cross-sectional area of the through-hole 116a in the embodiment is about to 4.73% of the area of the lower surface of the top plate 116, but below 10% is an acceptable range.

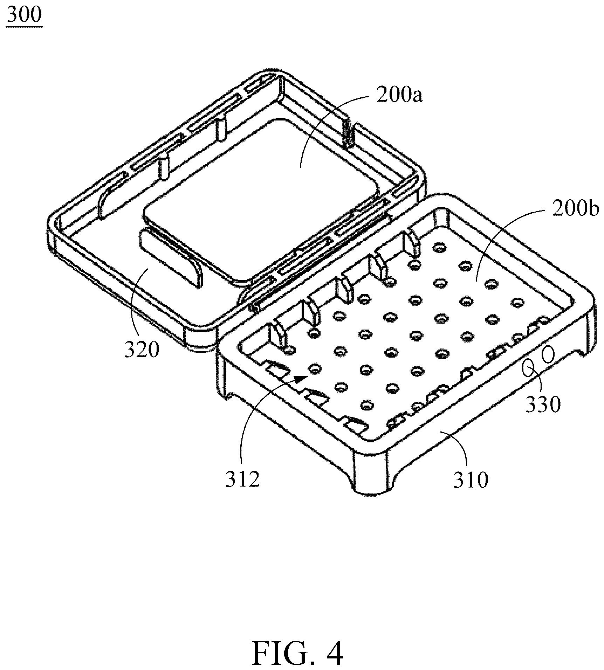

Refer to FIG. 4. As shown in figure, the temperature difference device 300 is operable in conjunction with the sperm sorting device 100 to facilitate sperm sorting and improve the overall convenience of the sperm sorting process. Specifically, the temperature difference device 300 includes a main body 310, a lid 320 and a controlling unit 330. The main body 310 is formed with a sorting device accommodating portion 312 configured to accommodate at least a portion of the sperm sorting device 100. The lid 320 is selectively openable relative to the main body 310. A temperature controlling component 200a for heating is disposed at the lower surface of the lid 320, and another temperature controlling component 200b for cooling is disposed at the bottom surface of the sorting device accommodating portion 312. On the other hand, the controlling unit 330 is exemplary to be a button electrically connected to the temperature controlling component 200 to set parameters such as continuation time. Furthermore, as shown in FIG. 3 and FIG. 4, an outer wall defining the lower chamber 121 of the lower portion 120 may be convex outwards with respect to other portions, and the sorting device accommodating portion 312 has a concaved portion configured to receive the outer wall. By the configuration, when the sperm sorting device 100 is accommodated in the sorting device accommodating portion 312, and the lid 320 is closed relative to the main body 310, the convex portion of the lower portion 120 will be engaged in the sorting device accommodating portion 312, so as to prevent the sperm sorting device 100 from vibrating. Moreover, user can operate the temperature controlling component 200a to heat the upper chamber 111, or operate the temperature controlling component 200b to dissipate heat from the lower chamber 121 through the controlling unit 330. The temperature difference required for sorting is thus obtained.

The specific steps of the sperm sorting method in the embodiment are illustrated below:

### Human sperm handling and sperm sorting

Human semen samples were obtained from donors after 3 days of sexual abstinence. Informed consent was obtained from each donor. After obtaining semen samples from the hospital, liquefied-semen samples were then analyzed by CASA (computer-assisted semen analysis). Subsequently, a semen sample was split into two fractions for sperm sorting in the presence and absence of temperature difference condition. Spermatozoa were separated from crude semen sample by the following process. Briefly, a semen sample was injected into the lower chamber 121 at the second side of the porous layer 130 through the injecting port 112; the buffer 140 (mHTF medium) is then injected into the upper chamber 111 at the first side of the porous layer 130. Whether to use the temperature controlling component 200 is decided according to the tested group. After disposing the unsorted sperms, the sorted sperms were collected after 15 to 30 minutes incubation, and analyzed by CASA.

### Immunofluorescence staining

To compare the quality of spermatozoa before and after sorting, approximately 2×10⁶ spermatozoa were centrifuged for 7 minutes and resuspended in PBS containing 4% of paraformaldehyde. After fixation, the samples were centrifuged and washed with PBS twice, and then resuspended in PBS at 200 µL. Fixed cells were then aliquoted and smeared onto a glass microscope slide and left to dry. The air-dried slides can be placed into storage at -80 °C or performed immunofluorescence staining directly. The assessment of sperm DNA fragmentation (sDF) in spermatozoa was evaluated by using with TUNEL assay.

### Vertical temperature difference establishment

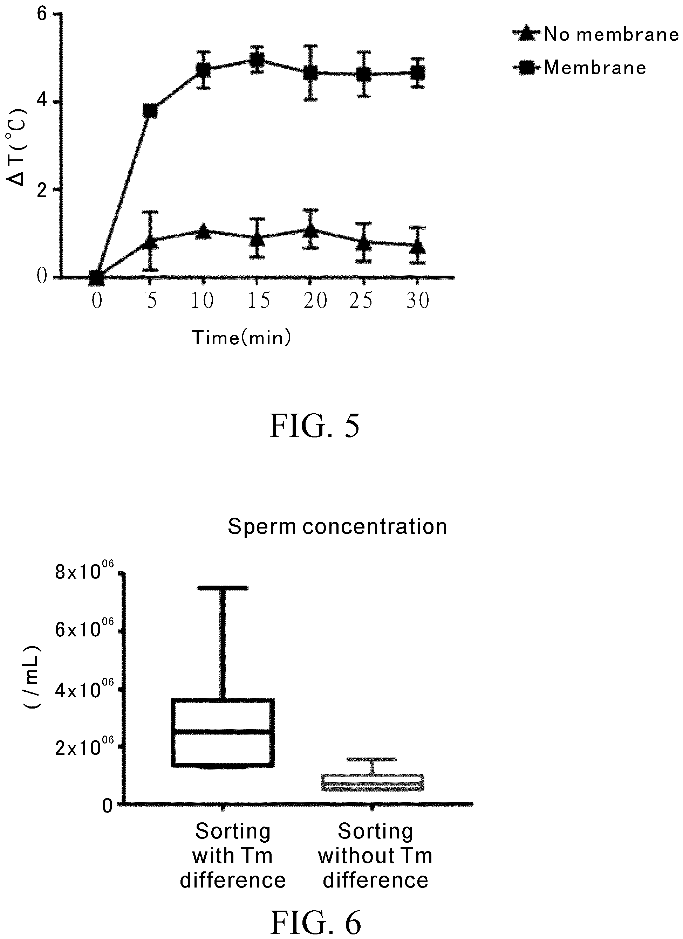

In some embodiments, the temperature difference may be formed between the upper chamber 111 and the lower chamber 121 through the temperature controlling component 200, and the temperature of the mild heat source and heat released by the temperature controlling component 200 were detected by an Infrared thermal imager (MT-4606, Proskit). It is noted that the PCTE membrane selected for the porous layer 130 not only allows motile sperms to travel from the lower chamber 121 to the upper chamber 111, but also has the direct benefit of enhancing the temperature difference. As shown in FIG. 5, the sperm sorting method with disposing the porous layer 130 in the embodiment forms an average temperature difference between the upper chamber 111 and the lower chamber 121 at the range of 3.80-4.97°C in 30 minutes, and an average temperature difference between the upper chamber 111 and the lower chamber 121 for a sperm sorting method without disposing the porous layer 130 is determined at the range of 0.73-1.10°C. The result shows that the average temperature difference enhanced by the sperm sorting method with the use of the porous layer 130 can be maintained at least 3°C. Besides, compared with the prior horizontal temperature difference system for spermatozoa isolation, the temperature difference can be achieved in a space of only 4 mm (4 mm for the upper chamber 111,4 mm for the lower chamber 121), and only a simple heat source is required. Therefore, the sperm sorting method in the embodiment is convenient and minimizes the sperm sorting device 100.

### Sperm motility, concentration, and isolation efficiency

The improvement of sperm sorting with a vertical temperature difference system was assessed in compared to sperm sorting without a vertical temperature difference system. The sperm concentration, motility as well as sperm parameters of the sperm collected through the collecting port 113 were analyzed by CASA. As shown in FIG. 6, sperm sorting utilizing vertical temperature difference exhibited an increase in total sperm counts compared to sperm sorting at a uniform temperature, with an increase of approximately 1.86- to 6- fold.

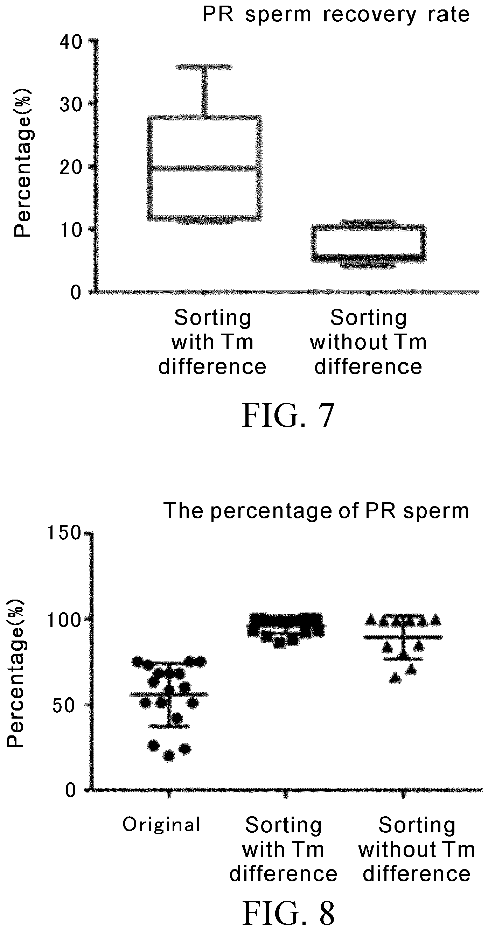

Refer to FIG. 7. The recovery rate of progressive sperms (PR sperms) was increased approximately two- to four-fold in the sperm sorting with vertical thermal difference as compared with control (sorting with uniform temperature). More importantly, as shown in FIG. 8, these results also demonstrated that the vertical thermal difference system provides a higher percentage of PR sperms (96±4.86%) as compared with the uniform temperature system (89.27±12.71%) and unsorted sample (54±16.84%).

### Sperm velocity analysis

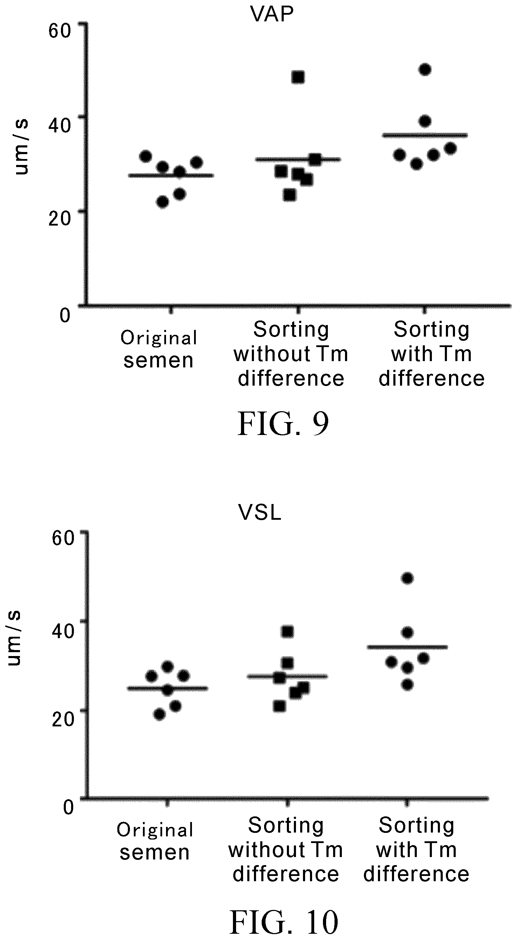

The sperms and moving paths of the sorted sperm were tracked by CASA, as shown in FIGS. 9-11. The thermotaxis-sorted sperm showed a series of velocity parameters significantly higher than that of the raw sperm sample. The values of VAP, VSL, and VCL tend to increase in the vertical temperature difference system, suggesting that sperms that responded to temperature difference possess better velocity parameters.

### Quality of thermotactic spermatozoa

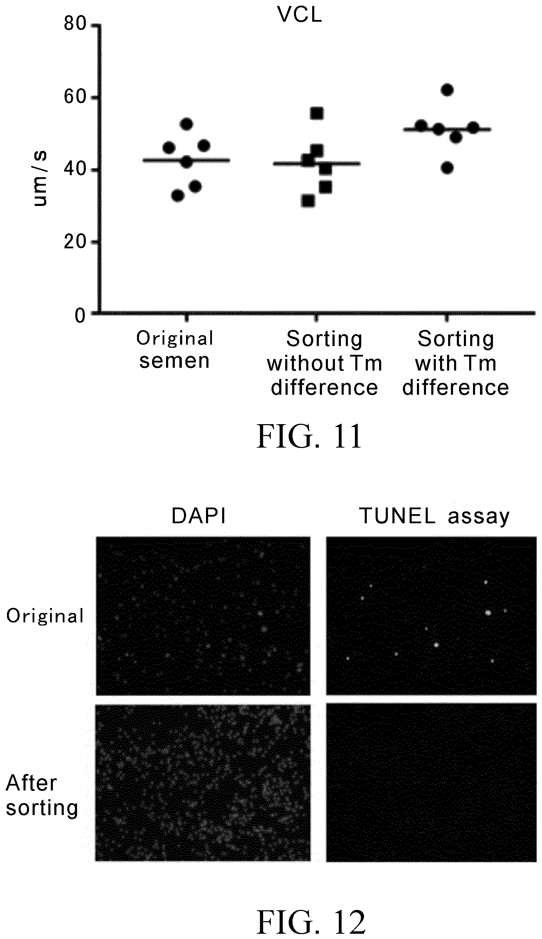

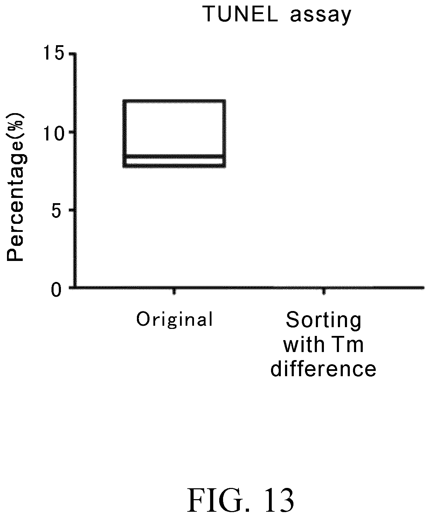

To assess the genetic quality of spermatozoa selected by vertical temperature difference device, we examined DNA fragmentation level after sorting with our vertical sorting system by TUNEL assay. As shown in FIG. 12 and FIG. 13, the TUNEL index in spermatozoa after being sorted by a vertical temperature difference system was reduced to almost 0 % as compared to the unsorted sample.

The disclosure imitates the avigation mechanism of spermatozoa in the female genital tract, it could be used as a tool in the ART setting to select the best spermatozoa as human spermatozoa after capacitation responds positively to a temperature difference.

While various aspects and embodiments have been disclosed herein, other aspects and embodiments will be apparent to those skilled in the art. The various aspects and embodiments disclosed herein are for purposes of illustration and are not intended to be limiting, with the true scope being indicated by the following claims, along with the full scope of equivalents to which such claims are entitled. It is also to be understood that the terminology used herein is for the purpose of describing particular embodiments only, and is not intended to be limiting.

## Claims

1. A sperm sorting device (100) adapted to collaborate with a temperature controlling component (200), **characterized by** comprising:
an upper portion (110) formed with an upper chamber (111);
a lower portion (120) formed with a lower chamber (121), wherein the upper chamber (111) and the lower chamber (121) are disposed vertically, and the temperature controlling component (200) is adapted to be disposed outside the upper portion (110) or the lower portion (120) to make the temperature of the upper chamber (111) higher than the lower chamber (121); and
a porous layer (130) disposed between the upper chamber (111) and the lower chamber (121), adapted to allow sperms to pass through from the lower chamber (121) to the upper chamber (111) and adapted to form a temperature difference between the upper chamber (111) and the lower chamber (121).

2. The sperm sorting device (100) according to claim 1, **characterized in that** the upper portion (110) comprises a top plate (116) disposed at a top side of the upper chamber (111), the temperature controlling component (200) is adapted to transfer heat uniformly through the top plate (116) to the upper chamber (111).

3. The sperm sorting device (100) according to claim 2, **characterized in that** the top plate (116) has a lower surface and is formed with a through-hole (116a) disposed at a higher end of the lower surface, and varied height difference is formed between the lower surface and a bottom of the upper chamber (111).

4. The sperm sorting device (100) according to claim 3, **characterized in that** the lower surface is an incline.

5. The sperm sorting device (100) according to claims 3 or 4, **characterized in that** a cross-sectional area of the through-hole (116a) is less than 10% of an area of the lower surface.

6. The sperm sorting device (100) according to anyone of claims 1-5, **characterized in that** the upper portion (110) is further formed with a collecting port (113) communicating with the upper chamber (111).

7. The sperm sorting device (100) according to anyone of claims 1-6, **characterized in that** the upper portion (110) is further formed with an injecting port (112) communicating with the lower chamber (121).

8. The sperm sorting device (100) according to anyone of claims 1-7, **characterized in that** a material of the porous layer (130) is selected from the group consisting of polycarbonate, polyvinyl alcohol, polypropylene, polytetrafluoroethylene, polystyrene, polyethylene terephthalate, polyvinylidene fluoride, polyamide, non-woven fabric and polyurethane.

9. The sperm sorting device (100) according to anyone of claims 1-8, **characterized in that** the porous layer (130) is formed with a plurality of holes, and pore sizes of the plurality of holes are 8-20 mm.
